# EUROPEAN PATENT SPECIFICATION

(11) **EP 2 796 871 B1**
(45) Date of publication and mention of the grant of the patent: **03.02.2016**
(21) Application number: 14163951.8
(22) Date of filing: 08.04.2014
(51) Int. Cl.: G01N 33/49, B01L 3/00

(54) **PARTICLE IMAGING UTILIZING A FILTER**
PARTIKELBILDGEBUNG UNTER VERWENDUNG EINES FILTERS
IMAGERIE DE PARTICULES UTILISANT UN FILTRE

(30) Priority: 24.04.2013 US 201313869880
(43) Date of publication of application: 29.10.2014
(73) Proprietor: Honeywell International Inc., Morris Plains, NJ 07950 (US)
(72) Inventor: Palaima, Elizabeth, Morristown, NJ 07962-2245 (US); Hoehn, Kylin, Morristown, NJ 07962-2245 (US); Burris, Steve, Morristown, NJ 07962-2245 (US); Seifried, Lynn, Morristown, NJ 07962-2245 (US); Wong, Pamela, Morristown, NJ 07962-2245 (US)
(74) Representative: Houghton, Mark Phillip

(56) References cited:
- EP-A2- 0 965 388
- WO-A1-03/100402
- DE-A1-102011 078 961

## Description

### Background

Particles, such as white blood cells are counted and differentiated for use in medical studies and diagnostic testing. Microfluidic structures have been used to isolate white blood cells on slides or in cuvettes. However, the slides and cuvettes have not provided an easy effective mechanism to quickly and accurately count and differentiate white blood cells.

DE102011078961 discloses a system for separating bodily fluid constituents comprising a filter arranged between two substrates in which a first cavity is arranged in one substrate to receive fluid on one side of the filter and a third channel is arranged to receive fluid passed transversely through the filter from the first cavity.

### Summary

A multiple layer test card including an input channel formed in a layer of the test card, an output channel formed in another layer of the test card, and a filter coupled between the input channel and the output channel to receive fluid from the input channel and collect particles such that the collected particles are optically visible proximate a first surface of the filter, wherein the input channel provides the fluid proximate a first portion of a length of the filter, and the output channel receives fluid passed over the filter proximate a second portion of the length of the filter, and the first and second portions are separated along the length of the filter.

A method includes providing a fluid containing particles of interest to a microfluidic input channel on a layer of a multiple layer laminated test card, moving the fluid through the input channel and over a filter on a filter layer of the test card, and collecting the particles of interest on the filter, wherein the input channel provides the fluid proximate a first portion of a length of the filter, and the output channel receives fluid passed over the filter proximate a second portion of the length of the filter, and the first and second portions are separated along the length of the filter.

A further method includes forming an input channel in an input layer of a multiple layer test card, forming a filter in a filter layer of the multiple layer test card, forming a waste channel in a output layer of the multiple layer test card, providing a capping layer for each of the input channel and waste channel, and attaching the layers together such that the input channel opens onto a first side of the filter proximate a first portion of a length of the filter and the waste channel opens onto a second side of the filter proximate a second portion of a length of the filter separated along the length of the filter from the first portion, such that particles in the fluid are collected on the filter as the fluid is moved over the filter.

### Brief Description of the Drawings

FIG. 1 is a top view representation of a card having a filter according to an example embodiment.
FIG. 2 is a side cross section representation of the card of FIG. 1 taken along lines 2-2 according to an example embodiment.
FIG. 3 is a flowchart representation of a method of using a test card according to an example embodiment.
FIG. 4 is a flowchart representation of a method of manufacturing a multiple layer test card according to an example embodiment.
FIG. 5 is an exploded view of an alternative cartridge having a filter according to an example embodiment.
FIG. 6 is an exploded view of a filter assembly according to an example embodiment.
FIG. 7 is a top view of the filter of FIG. 6 that illustrates example dimensions of the filter

### Detailed Description

In the following description, reference is made to the accompanying drawings that form a part hereof, and in which is shown by way of illustration specific embodiments which may be practiced. These embodiments are described in sufficient detail to enable those skilled in the art to practice the invention, and it is to be understood that other embodiments may be utilized and that structural, logical and electrical changes may be made without departing from the scope of the present invention. The following description of example embodiments is, therefore, not to be taken in a limited sense, and the scope of the present invention is defined by the appended claims.

White cells are important to immune system and function. Both white cell counts and differentiation are used in medical studies. A filter allows one to stain and image a liquid sample of varying sizes containing white cells for further analysis such as differentiation of a sample utilizing a relatively small area. The filter provides additional flexibility of use to the system, and may also be used to collect and image many different types of particles of interest.

In various embodiments a screen or filter is used increase the concentration of particles of interest. The filter is then ready to be viewed to facilitate detection of a small number of particles from varying sample sizes, viewing only a small surface area. Furthermore since the particles can be stained on or before the filter they can be easily detected and possibility differentiated.

In some embodiments, the filter material may be a polycarbonate filter or other non-fibrous filter material. The filter provides a surface that can be dried which provides an opportunity for fixation and staining if desired. Previous attempts to provide an automated system to image white cells utilized cuvettes or slides. These are not as nearly as adaptable as the filter for use in an automated system with varying sample sizes.

Microfluidic cards may be assembled using filters and different combination of materials. After constructing the cards, in one embodiment, blood is pulled or pushed into a sample input channel to one side of the filter combinations mentioned above and then applied over the filter.

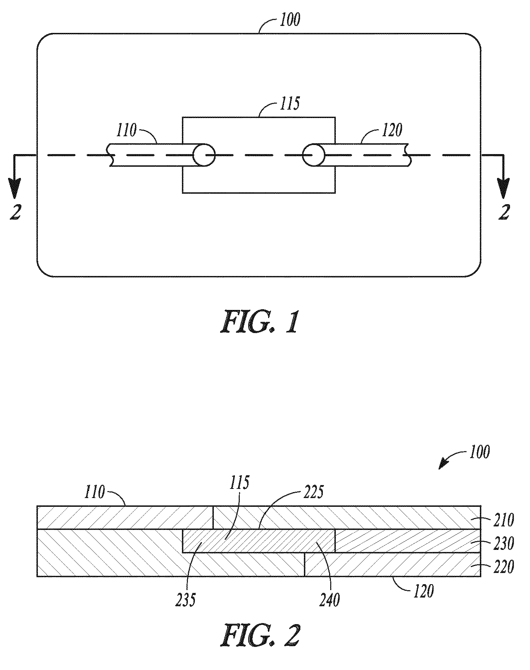

FIG. 1 is a block diagram planar view representation of a multiple layer test card 100. FIG. 2 is a cross section of the test card 100 taken along lines 2-2 in FIG. 1, with consistent number of elements. The figures are not to scale. In some embodiments, the test card 100 contains many layers of a transparent material such as PET or acrylic or other suitable material that can be patterned with various liquid fluid transport features. The card 100 in some embodiments may be used to perform one or more blood tests utilizing a small volume of blood. The blood or other liquid to be tested, may be transported via one or more layers of the test card, and prepared for analysis by a test instrument into which the card is inserted A combination of LEDs, lasers, and other light sources may be used to illuminate the sample.

In one embodiment, card 100 includes an input channel 110 formed in an input layer 210 of the test card. A waste channel 120 is formed in a waste layer 220 of the test card 100. A filter 115 is coupled between the input channel and the waste channel to receive fluid from the input channel and collect particles such as white blood cells such that the collected particles are optically visible proximate a first surface 225 of the filter 115. The filter 115 may be deposited within a cut out area of a filter layer 230, or may be simply sandwiched between the input layer 210 and waste layer 220 in various embodiments. Further layers may provide a capping function for the channels.

The input channel 110 provides the fluid proximate a first portion 235 of a length of the filter 115, and the waste channel receives fluid passed through the filter 115 proximate a second portion 240 of the length of the filter. The first and second portions of the length of the filter may be separated along the length of the filter. In further embodiments, the first and second portions overlap.

In one embodiment, the filter is optically visible at least along a portion of the filter between the first and second portions of the filter. The layers of the card may be transparent to facilitate visibility of the filter through the layers.

In one embodiment, the filter or the channel before the filter 115 includes a stain to stain the collected particles such as white blood cells. The stain may be formed of acridine orange, other nucleic acid stain, or stain that differentiates white cells. The filter may have a pore size of between 2 and 5 µm in some embodiments, and larger or smaller pore sizes in further embodiments. The filter is formed of non-fibrous filter material. One example filter is a polycarbonate filter.

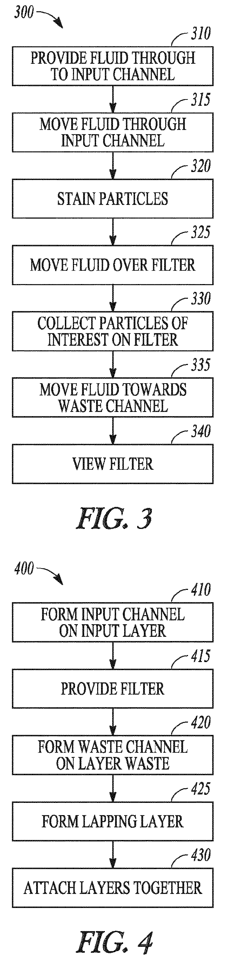

FIG. 3 is a flowchart of a method 300 of using a test card. At 310, fluid containing particles of interest are provided to a microfluidic input channel on a layer of a multiple layer test card. At 315, the fluid is moved through the input channel and particles are stained at 320. The fluid then moves over a filter at 325 on a filter layer of the test card. At 330, particles of interest are collected on the filter. The fluid is moved at 335 from the filter towards a waste channel in a waste channel layer of the test card.

In one embodiment, the fluid is moved via negative or positive pressure provided via a test system in which the card has been inserted. The filter or input channel before the filter may contain stain, such that the particles of interest that are collected on the filter are stained at or before 235 to make them more easily visible. In some embodiments, the particles of interest are stained by acridine orange. The acridine orange may be contained on the filter or the channel before the filter. The fluid in one embodiment comprises blood and the particles of interest comprise white blood cells. The filter may be viewed or imaged at 340 to provide an image from which differentiation of cells may be performed, either via image analysis or by human analysis.

FIG. 4 is a flowchart illustrating a method 400 of building a card. At 410, an input channel is formed in an input layer of a multiple layer test card. A filter is formed in a filter layer of the multiple layer test card at 415. Alternatively, a filter is provided without a corresponding filter layer. At 420, a waste channel is formed in a waste layer of the multiple layer test card. In one embodiment, the channels are cut from the layers via a laser. A capping layer is formed for each of the input channel and waste channel at 425 by providing a layer that has material corresponding to channels to cover the channels when the layers are assembled together. At 430, the layers are attached together such that the input channel opens onto a first side of the filter and the waste channel opens onto a second side of the filter opposite the first side such that particles in the fluid are collected on the filter as the fluid is moved over the filter. The layers may be attached by a laminating process, double sided adhesive layers having corresponding cut out areas to allow fluid to move between adjacent layers where intended, or other mechanisms, such as clamping between stiff layers. Some cards or layers to be attached may also be made via one or more injection molding processes.

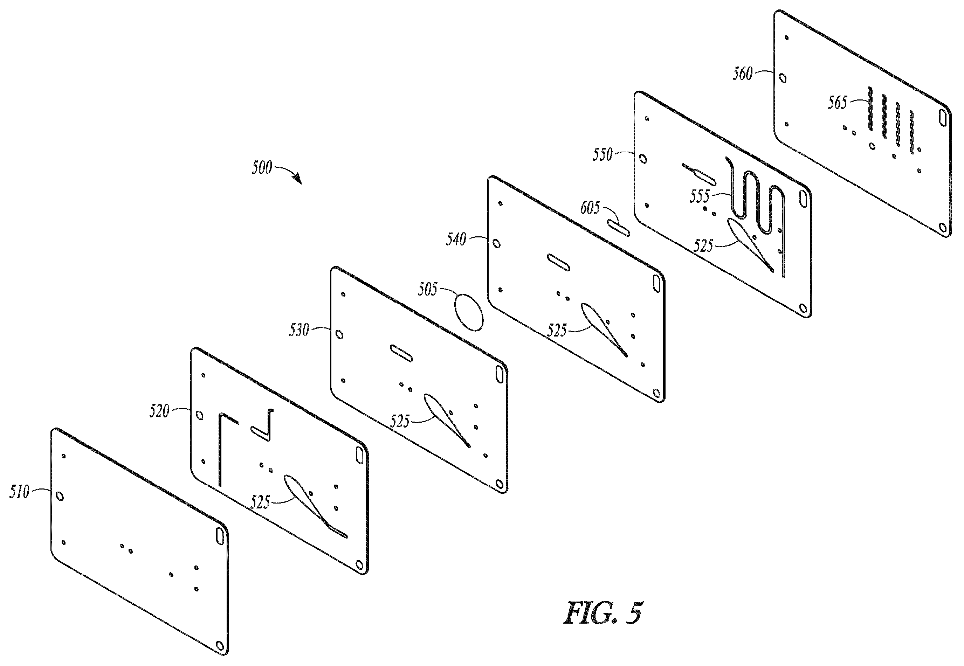

FIG. 5 is an exploded view of an alternative cartridge 500 having a filter 505 according to an example embodiment. Six layers, 510, 520, 530, 540, 550, and 560 are shown with various features that when assembled provide a fluid having particles, such as white blood cells to the filter 505, which is sandwiched between layers 530 and 540. In one embodiment, blood is received in a sample well 525, that has a depth of four layers 520, 530, 540, 550. The fluid proceeds through a serpentine path 555 as seen on layer 550. Remaining white blood cells are transported to a first side of filter 505, which collects the particles. Remaining fluid moves over the filter to one or more waste channels. In this embodiment, the filter 505 is simply sandwiched between two layers.

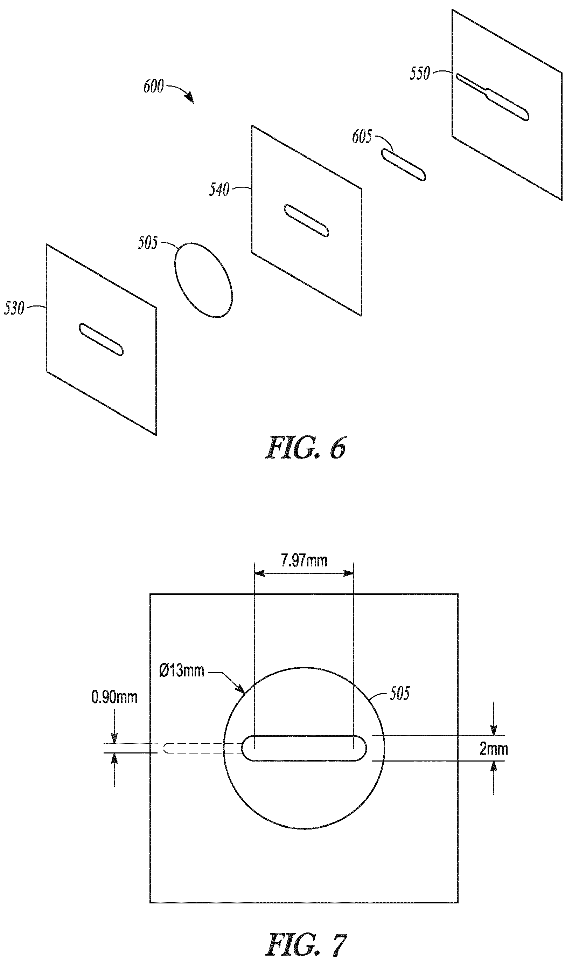

FIG. 6 is an exploded view of a filter assembly according to an example embodiment. An absorbent pad 605 may be included and is shown between layers 540 and 550. Pad 605 may be positioned to support the filter and better present the particles in the openings of the layers adjacent the filter 505. FIG. 7 is a top view of the filter 505 that illustrates example dimensions of the filter 505 and channels on either side in adjacent layers that sandwich the filter.

### Examples:

1. A multiple layer test card comprising:
   an input channel formed in a first layer of the test card;
   an output channel formed in a second layer of the test card; and
   a filter coupled between the input and output channels to receive fluid from the input channel and collect particles such that the collected particles are optically visible proximate a first surface of the filter.
2. The multiple layer test card of example 1 wherein the filter or the input channel includes a stain to stain the collected particles comprising white blood cells.
3. The multiple layer test card of example 2 wherein the stain comprises acridine orange.
4. The multiple layer test card of any of examples 2-3 wherein the stain comprises a nucleic acid stain, a pH sensitive stain, or a stain that differentiates white cells.
5. The multiple layer test card of any of examples 1-4 wherein the filter has a pore size of between 2 and 5 µm.
6. The multiple layer test card of example 5 wherein the input channel provides the fluid proximate a first portion of a length of the filter, and the output channel receives fluid passed over the filter proximate a second portion of the length of the filter.
7. The multiple layer test card of example 6 wherein the first and second portions of the length of the filter are separated along the length of the filter.
8. The multiple layer test card of example 7 wherein the filter is optically visible at least along a portion of the filter between the first and second portions of the filter.
9. The multiple layer test card of any of examples 1-8 wherein the filter is formed of non-fibrous filter material.
10. The multiple layer test card of any of examples 1-9 wherein the filter comprises a polycarbonate filter.
11. The multiple layer test card of any of examples 1-10 and further comprising an absorbent pad disposed to support the filter.
12. A method comprising:
   providing a fluid containing particles of interest to a microfluidic input channel on a layer of a multiple layer laminated test card;
   moving the fluid through the input channel and over a filter on a filter layer of the test card;
   collecting the particles of interest on the filter; and
   moving the fluid from the filter towards a waste channel in a waste channel layer of the test card.
13. The method of example 12 wherein moving the fluid is moved via negative or positive pressure.
14. The method of any of examples 12-13 and further comprising staining the particles of interest that are collected on the filter.
15. The method of example 14 wherein the particles of interest are stained by acridine orange.
16. The method of example 15 wherein the acridine orange is contained in at least one aspect of the input channel and or on the filter.
17. The method of any of examples 12-16 wherein the fluid comprises blood and the particles of interest comprise white blood cells.
18. The method of any of examples 12-17 wherein the filter has a pore size of between 2 and 5 µm and wherein the input channel provides the fluid proximate a first portion of a length of the filter, and the waste channel receives fluid passed over the filter proximate a second portion of the length of the filter.
19. The method of any of examples 12-18 wherein the filter comprises a polycarbonate filter.
20. A method comprising:
   forming an input channel in an input layer of a multiple layer test card;
   forming a filter in a filter layer of the multiple layer test card;
   forming a waste channel in a waste layer of the multiple layer test card;
   providing a capping layer for each of the input channel and waste channel; and
   attaching the layers together such that the input channel opens onto a first side of the filter and the waste channel opens onto a second side of the filter opposite the first side such that particles in the fluid are collected on the filter as the fluid is moved over the filter.
21. The method of example 20 wherein forming the channels comprises cutting the channels in each layer with a laser.

Although a few embodiments have been described in detail above, other modifications are possible. For example, the logic flows depicted in the figures do not require the particular order shown, or sequential order, to achieve desirable results. Other steps may be provided, or steps may be eliminated, from the described flows, and other components may be added to, or removed from, the described systems. Other embodiments may be within the scope of the following claims.

## Claims

1. A multiple layer test card (100, 500) comprising:
an input channel (110) formed in a layer of the test card;
an output channel (120) formed in another layer of the test card; and
a filter (115, 505) coupled between the input channel (110) and the output channel (120) to receive fluid from the input channel (110) and collect particles, such that the collected particles are optically visible proximate a first surface (225) of the filter (115, 505);
wherein the input channel (110) provides the fluid proximate a first portion (235) of a length of the filter (115, 505), and the output channel (120) receives fluid passed over the filter (115, 505) proximate a second portion (240) of the length of the filter (115, 505), and the first and second portions (235, 240) are separated along the length of the filter (115, 505).

2. The multiple layer test card (100, 500) of claim 1, wherein the filter (115, 505) or the input channel (110) before the filter (115, 505) includes a stain to stain the particles comprising collected white blood cells.

3. The multiple layer test card (100, 500) of claim 2, wherein the stain comprises acridine orange.

4. The multiple layer test card (100, 500) of claim 2, wherein the stain comprises a nucleic acid stain, a pH sensitive stain, or a stain that differentiates white cells.

5. The multiple layer test card (100, 500) of any one of claims 1-4, wherein the filter (115, 505) is formed of non-fibrous filter material.

6. The multiple layer test card (100, 500) of any one of claims 1-4, further comprising an absorbent support pad (605) disposed to support the filter (115, 505) and better present particles to the filter (115, 505).

7. A method comprising:
providing (310) a fluid containing particles of interest to a microfluidic input channel (110) on a layer (210) of a multiple layer laminated test card (100);
moving (315) the fluid through the input channel (110) and over a filter (115, 505) on a filter layer of the test card (100);
collecting (330) the particles of interest in the filter (115, 505); and
moving (335) the fluid from the filter (115, 505) towards a waste channel (120) in a waste channel layer (220) of the test card (100);
wherein the input channel (110) provides the fluid proximate a first portion (235) of a length of the filter (115, 505), and the waste channel (120) receives fluid passed through the filter (115, 505) proximate a second portion (240) of the length of the filter (115, 505) and the first and second portions (235, 240) are separated along the length of the filter (115, 505).

8. The method of claim 7, and further comprising staining (320) the particles of interest that are collected on the filter (115, 505).

9. The method of claim 8, wherein the particles of interest are stained by acridine orange.

10. The method of claim 9, wherein the acridine orange is contained in at least one of the input channel (110) and the filter (115, 505).

11. The method of any one of claims 7-10 wherein the filter (115, 505) has a pore size of between 2 and 5 µm.

12. A method comprising:
forming (410) an input channel (110) in an input layer (210) of a multiple layer test card (100);
forming (415) a filter (115, 505) in a filter layer (230) of the multiple layer test card (100);
forming (420) a waste channel (120) in a waste layer (220) of the multiple layer test card (100);
providing (425) a capping layer for each of the input channel (110) and waste channel (120); and
attaching (430) the layers together such that the input channel (110) opens onto a first side of the filter (115, 505) proximate a first portion (235) of a length of the filter (115, 505) and the waste channel (120) opens onto a second side of the filter (115, 505) proximate a second portion (240) of the length of the filter (115, 505) separated along the length of the filter (115, 505) from the first portion (235), such that particles in the fluid are collected on the filter (115, 505) as the fluid is moved over the filter (115, 505).

13. The method of claim 12, wherein forming (410, 420) the channels (110, 120) comprises cutting the channels (110, 120) in each layer with a laser.

## Patentansprüche

1. Mehrschicht-Testkarte (100, 500), umfassend:
einen Eingangskanal (110), der in einer Schicht der Testkarte ausgebildet ist; einen Ausgangskanal (120), der in einer anderen Schicht der Testkarte ausgebildet ist; und einen Filter (115, 505), der zwischen dem Eingangskanal (110) und dem Ausgangskanal (120) gekoppelt ist, um Fluid von dem Eingangskanal (110) zu erhalten und Partikel zu sammeln, sodass die gesammelten Partikel optisch in der Nähe einer ersten Oberfläche (225) des Filters (115, 505) sichtbar sind;
wobei der Eingangskanal (110) das Fluid in der Nähe eines ersten Abschnitts (235) einer Länge des Filters (115, 505) bereitstellt und der Ausgangskanal (120) Fluid, das über den Filter (115, 505) weitergegeben wird, in der Nähe eines zweiten Abschnitts (240) der Länge des Filters (115, 505) aufnimmt und der erste und der zweite Abschnitt (235, 240) entlang der Länge des Filters (115, 505) getrennt sind.

2. Mehrschicht-Testkarte (100, 500) nach Anspruch 1, wobei der Filter (115, 505) oder der Eingangskanal (110) vor dem Filter (115, 505) ein Färbemittel aufweist, um die Partikel zu färben, die gesammelte weiße Blutzellen umfassen.

3. Mehrschicht-Testkarte (100, 500) nach Anspruch 2, wobei das Färbemittel Acridinorange umfasst.

4. Mehrschicht-Testkarte (100, 500) nach Anspruch 2, wobei das Färbemittel ein Nukleinsäure-Färbemittel, ein pH-empfindliches Färbemittel oder ein Färbemittel umfasst, das weiße Zellen differenziert.

5. Mehrschicht-Testkarte (100, 500) nach einem der Ansprüche 1 bis 4, wobei der Filter (115, 505) aus nicht-faserigem Filtermaterial ausgebildet ist.

6. Mehrschicht-Testkarte (100, 500) nach einem der Ansprüche 1 bis 4, weiterhin umfassend ein absorbierendes Stützkissen (605), das angeordnet ist, um den Filter (115, 505) abzustützen und die Partikel dem Filter (115, 505) besser vorzulegen.

7. Verfahren, umfassend:
Bereitstellen (310) eines Fluids, das Partikel von Interesse für einen mikrofluidischen Eingangskanal (110) auf einer Schicht (210) einer mehrschichtigen Testkarte (100) enthält;
Bewegen (315) des Fluids durch den Eingangskanal (110) und über einen Filter (115, 505) auf einer Filterschicht der Testkarte (100);
Sammeln (330) der Partikel von Interesse in dem Filter (115, 505); und Bewegen (335) des Fluids von dem Filter (115, 505) zu einem Abführkanal (120) in einer Abführkanalschicht (220) der Testkarte (100);
wobei der Eingangskanal (110) das Fluid in der Nähe eines ersten Abschnitts (235) einer Länge des Filters (115, 505) bereitstellt und der Abführkanal (120) Fluid durch den Filter (115, 505) in der Nähe eines zweiten Abschnitts (240) der Länge des Filters (115, 505) aufnimmt und der erste und der zweite Abschnitt (235, 240) entlang der Länge des Filters (115, 505) getrennt sind.

8. Verfahren nach Anspruch 7, ferner umfassend das Färben (320) der Partikel von Interesse, die auf dem Filter (115, 505) gesammelt werden.

9. Verfahren nach Anspruch 8, wobei die Partikel von Interesse in Acridinorange gefärbt werden.

10. Verfahren nach Anspruch 9, wobei Acridinorange mindestens entweder im Eingangskanal (110) oder dem Filter (115, 505) enthalten ist.

11. Verfahren nach einem der Ansprüche 7 bis 10, wobei der Filter (115, 505) eine Porengröße zwischen 2 und 5 µm aufweist.

12. Verfahren, umfassend:
Bilden (410) eines Eingangskanals (110) in einer Eingangsschicht (210) einer Mehrschicht-Testkarte (100) ;
Bilden (415) eines Filters (115, 505) in einer Filterschicht (230) der Mehrschicht-Testkarte (100);
Bilden (420) eines Abführkanals (120) in einer Abführschicht (220) der Mehrschicht-Testkarte(100);
Bereitstellen (425) einer Deckschicht für jeden Eingangskanal (110) und Abführkanal (120); und
Befestigen (430) der Schichten aneinander, sodass der Eingangskanal (110) auf einer ersten Seite des Filters (115, 505) nahe eines ersten Abschnitts (235) einer Länge des Filters (115, 505) öffnet und der Abführkanal (120) auf einer zweiten Seite des Filters (115, 505) nahe eines zweiten Abschnitts (240) der Länge des Filters (115, 505) öffnet, der entlang der Länge des Filters (115, 505) vom ersten Abschnitt (235) getrennt ist, sodass die Partikel in dem Fluid auf dem Filter (115, 505) gesammelt werden, wenn das Fluid über den Filter (115, 505) bewegt wird.

13. Verfahren nach Anspruch 12, wobei das Bilden (410, 420) der Kanäle (110, 120) das Schneiden der Kanäle (110, 120) in jeder Schicht mit einem Laser umfasst.

## Revendications

1. Carte de test multicouches (100, 500) comprenant :
un canal d'entrée (110) formé dans une couche de la carte de test ;
un canal de sortie (120) formé dans une autre couche de la carte de test ; et
un filtre (115, 505) raccordé entre le canal d'entrée (110) et le canal de sortie (120) pour recevoir un fluide en provenance du canal d'entrée (110) et collecter des particules, de telle sorte que les particules collectées soient optiquement visibles à proximité d'une première surface (225) du filtre (115, 505) ;
dans laquelle le canal d'entrée (110) amène le fluide à proximité d'une première partie (235) d'une longueur du filtre (115, 505), et le canal de sortie (120) reçoit le fluide qui est passé dans le filtre (115, 505) à proximité d'une deuxième partie (240) de la longueur du filtre (115, 505), et les première et deuxième parties (235, 240) sont séparées le long de la longueur du filtre (115, 505).

2. Carte de test multicouches (100, 500) selon la revendication 1, dans laquelle le filtre (115, 505) ou le canal d'entrée (110) situé avant le filtre (115, 505) contient un colorant pour colorer les particules comprenant des leucocytes collectés.

3. Carte de test multicouches (100, 500) selon la revendication 2, dans laquelle le colorant comprend de l'orange d'acridine.

4. Carte de test multicouches (100, 500) selon la revendication 2, dans laquelle le colorant comprend un colorant d'acide nucléique, un colorant sensible au pH, ou un colorant qui différencie les leucocytes.

5. Carte de test multicouches (100, 500) selon l'une quelconque des revendications 1 à 4, dans laquelle le filtre (115, 505) est formé d'un matériau filtrant non fibreux.

6. Carte de test multicouches (100, 500) selon l'une quelconque des revendications 1 à 4, comprenant en outre un matelas de support absorbant (605) disposé de façon à supporter le filtre (115, 505) et mieux présenter les particules au filtre (115, 505).

7. Procédé comprenant :
le fait d'amener (310) un fluide contenant des particules d'intérêt à un canal d'entrée microfluidique (110) sur une couche (210) d'une carte de test multicouches stratifiée (100) ;
le fait de faire passer (315) le fluide dans le canal d'entrée (110) et dans un filtre (115, 505) sur une couche filtrante de la carte de test (100) ;
la collecte (330) des particules d'intérêt dans le filtre (115, 505) ; et
le fait d'amener (335) le fluide du filtre (115, 505) vers un canal de transport de déchets (120) dans une couche de canal de transport de déchets (220) de la carte de test (100) ;
dans lequel le canal d'entrée (110) amène le fluide à proximité d'une première partie (235) d'une longueur du filtre (115, 505), et le canal de transport de déchets (120) reçoit le fluide qui est passé dans le filtre (115, 505) à proximité d'une deuxième partie (240) le long de la longueur du filtre (115, 505), et les première et deuxième parties (235, 240) sont séparées le long de la longueur du filtre (115, 505).

8. Procédé selon la revendication 7, et comprenant en outre la coloration (320) des particules d'intérêt qui sont collectées sur le filtre (115, 505).

9. Procédé selon la revendication 8, dans lequel les particules d'intérêt sont colorées par de l'orange d'acridine.

10. Procédé selon la revendication 9, dans lequel l'orange d'acridine est présent dans au moins l'un du canal d'entrée (110) et du filtre (115, 505).

11. Procédé selon l'une quelconque des revendications 7 à 10, dans lequel le filtre (115, 505) a une taille de pore de 2 µm à 5 µm.

12. Procédé comprenant :
la formation (410) d'un canal d'entrée (110) dans une couche d'entrée (210) d'une carte de test multicouches (100) ;
la formation (415) d'un filtre (115, 505) dans une couche filtrante (230) de la carte de test multicouches (100) ;
la formation (420) d'un canal de transport de déchets (120) dans une couche de transport de déchets (220) de la carte de test multicouches (100) ;
la formation (425) d'une couche de recouvrement pour chacun du canal d'entrée (110) et du canal de transport de déchets (120) ; et
la fixation (430) des couches les unes aux autres de telle sorte que le canal d'entrée (110) s'ouvre sur un premier côté du filtre (115, 505) à proximité d'une première partie (235) d'une longueur du filtre (115, 505) et que le canal de transport de déchets (120) s'ouvre sur un deuxième côté du filtre (115, 505) à proximité d'une deuxième partie (240) de la longueur du filtre (115, 505) séparée le long de la longueur du filtre (115, 505) de la première partie (235), de telle sorte que des particules présentes dans le fluide soient collectées sur le filtre (115, 505) au fur et à mesure que le fluide passe dans le filtre (115, 505).

13. Procédé selon la revendication 12, dans lequel la formation (410, 420) des canaux (110, 120) comprend le découpage des canaux dans chaque couche (110, 120) avec un laser.
